# EUROPÄISCHE PATENTSCHRIFT

(11) **EP 0 587 090 B1**
(45) Veröffentlichungstag und Bekanntmachung des Hinweises auf die Patenterteilung: **22.01.1997**
(21) Anmeldenummer: 93114270.7
(22) Anmeldetag: 06.09.1993
(51) Int. Cl.: G01N 33/86, G01N 33/92, G01N 33/96

(54) **Verfahren zur Bestimmung von Thrombose verursachenden Lupus-anticoagulant-Antikörpern**
Method for determination of thrombosis causing lupus anticoagulant antibodies
Méthode pour la détermination des anticorps de lupus anticoagulant qui causent la thrombose

(30) Priorität: 08.09.1992 DE 4229933
(43) Veröffentlichungstag der Anmeldung: 16.03.1994
(73) Patentinhaber: Max-Planck-Gesellschaft zur Förderung der Wissenschaften e.V., D-37073 Göttingen (DE)
(72) Erfinder: Müller-Berghaus, Gert, Prof. Dr., D-35398 Giessen (DE); Pötzsch, Bernd, Dr. med., D-35390 Giessen (DE); Seelig, Christoph, D-35614 Asslar (DE)
(74) Vertreter: Böhm, Brigitte, Dipl.-Chem. Dr.

(56) Entgegenhaltungen:
- WO-A-91/01382
- WO-A-91/02812
- WO-A-92/10586
- WO-A-93/10261
- DATABASE WPI Section Ch, Week 9334, Derwent Publications Ltd., London, GB; Class B04, AN 93-267048 'In-vitro lupus anticoagulant determin. - comprises adding phospholipid, calcium ion and protein C-type activating substance in sample blood sample and determining coagulation time' & JP-A-5 180 835 (SRL KK) 23. Juli 1993
- BRITISH JOURNAL OF HAEMATOLOGY Bd. 76, Nr. 1 , September 1990 , OXFORD, GB Seiten 101 - 107 R.G. MALIA, S. KITCHEN, M. GREAVES AND F.E. PRESTON 'Inhibition of activated protein C and its cofactor protein S by antiphospholipid antibodies'
- THROMBOSIS AND HAEMOSTASIS Bd. 53, Nr. 1 , 18. Februar 1985 , STUTTGART, DE Seiten 15 - 18 THOMAS EXNER 'Similar Mechanism of Various Lupus anticoagulants'
- sample blood sample and determining coagulation time' & JP- A-5 180 835 (SRL KK) 23. Juli 1993

## Beschreibung

Die Erfindung betrifft ein Verfahren zur Bestimmung von LA-Antikörpern ("Lupus anticoagulant") im Blut, Plasma, Plasmafraktionen und Gewebeextrakten über deren Beeinflussung der Phospholipid-abhängigen gerinnungshemmenden Aktivität ("anticoagulant activity", antikoagulatorischen Aktivität) von aktiviertem Protein C (APC). Das erfindungsgemäße Verfahren kann angewendet werden, um bestimmte Prädispositionen oder Erkrankungen zu erkennen, einen Krankheitsverlauf zu überwachen oder eine Therapie zu kontrollieren.

Antiphospholipid-Antikörper (aPL) sind Autoantikörper, die bei Personen im Zusammenhang mit arteriellen und/oder venösen Verschlüssen, Thrombozytopenie und/oder spontanen Aborten auftreten (Übersichten: Lechner, K., Pabinger-Fasching, I., Haemostasis 15 (1985), 254-262; Branch, D.W. et al., N.Engl. J.Med. 313 (1985), 1322-1326; Exner, T., Thromb.Haemostas. 53 (1985), 15-18). aPL lassen sich durch verschiedene Tests nachweisen, z.B. durch einen ELISA unter Verwendung verschiedener Phospholipide als Antigen oder durch konventionelle Flockungstests. Diejenigen aPL, die durch Gerinnungstests nachgewiesen werden, bezeichnet man als LA-Antikörper ("Lupus anticoagulant"). LA-Antikörper interferieren mit den Phospholipid-abhängigen Gerinnungsschritten und verlängern deshalb die Gerinnungszeiten verschiedener Tests, ohne die Aktivität einzelner Gerinnungsfaktoren zu hemmen. Sie stellen aber keine einheitliche Gruppe von Autoantikörpern gegen ein ausreichend definiertes Antigen dar. Malia et al., British Journal of Haematology 76 (1990), 101-107 erwähnen eine mögliche Verbindung zwischen Antiphospholipid-Antikörpern und Thromboserisiko. Die Untersuchungen, welche zu diesem Ergebnis geführt haben, wurden mit aus Patientenplasma isolierten IgG-Fraktionen unter Zusatz von sowohl gereinigtem aktiviertem Protein C als auch Protein S und Phosphatidylserin und Phosphatidylcholin durchgeführt. Es wurde dabei auf einen inhibitorischen Effekt von Antiphospholipid-Antikörpern auf Komplexe von aktiviertem Protein C und Protein S geschlossen.

Es kann jedoch aufgrund der bisher zur Verfügung stehenden Tests nicht mit für die klinische Anwendung ausreichender Bestimmtheit und Schnelligkeit vorhergesagt werden, ob bei einem Patienten mit nachgewiesenem aPL tatsächlich auch ein Thromboserisiko vorliegt (Übersichten: Triplett, D.A., Sem.Thromb.Hemostas. 16 (1990), 182-192; Jouhikainen, T. et al., Blood Coagul.Fibrinol.3 (1992) 407-414).

Die WO 93/10261 beschreibt ein Verfahren zur Diagnose von Blutgerinnungsstörungen, wobei neben anderen Testreagenzien aktiviertes Protein C eingesetzt wird. Weiterhin wird in der WO 91/01382 ein Testverfahren zur Bestimmung der funktionellen Aktivität von Protein C bzw. Protein S offenbart. Der Probe wird dabei das Gerinnungsenzym FIXₐ zugegeben, worauf durch Messung der Thrombinmenge der Anteil an funktionell aktivem Protein S bzw. Protein C bestimmt werden kann. Die Bestimmung von LA-Antikörpern wird jedoch nicht in Betracht gezogen.

Mehrere Verfahren zur Bestimmung von LA-Antikörpern sind bekannt:
a. Bestimmung der Rekalzifizierungszeit (Übersicht: Rosner, E. et al., Thromb.Haemostas. 57 (1987), 144-147). Diese Methode hat eine niedrige Sensitivität und Spezifität.
b. Bestimmung der aktivierten partiellen Thromboplastinzeit unter Verwendung unterschiedlicher Aktivatoren und Phospholipide (Übersicht: Hemostasis Committee of the "Societe Francaise de Biologie Clinique", Thromb.Res. 66 (1992), 349-364). Sensitivität und Spezifität dieses Tests sind sehr niedrig.
c. Bestimmung der Kaolin-Gerinnungszeit bzw. Kaolin-Gerinnungszeit-Index (KCT) (Übersicht: Exner (1985) supra). Diese Tests haben sich als relativ gut zum Nachweis eines "Lupus anticoagulant" bewährt. Eine Korrelation zwischen dem Auftreten einer Thrombose und dem positiven Nachweis des mit diesem Test zu erfassenden "Lupus anticoagulant" besteht nicht.
d. Bestimmung über Dilute Russell's Viper Venom Time (Übersichten: Hemostasis Committee of the Societe Francaise de Biologie Clinique (1992) supra; Jouhikainen et al. (1992) supra). Mit diesem Test läßt sich mit einer relativ hohen Sensitivität ein "Lupus anticoagulant" nachweisen, jedoch besteht keine Korrelation zwischen einem positiven Testergebnis und einer Thrombosegefährdung.
e. Thrombozyten-Neutralisations-Test (Übersichten: Hemostasis Committee of the Societe Francaise de Biologie Clinique (1992) supra; Lazarchick, J. et al., Arch. Pathol.Lab.Med. 113 (1989), 177-180). Mit diesem Test lassen sich nur Autoantikörper nachweisen, deren gerinnungshemmende Aktivität in vitro durch thrombozytäre Phospholipide neutralisiert werden kann. Eine Korrelation zwischen einem positiven Nachweis des "Lupus antikoagulant" und einer Thrombosegefährdung besteht nicht.

Die oben aufgeführten Methoden sind mehr oder weniger unpräzise und haben vor allen Dingen den Nachteil, daß sie nur bestimmte Funktionen der LA-Antikörper erfassen, aber keine Aussage über eine Thrombosegefährdung liefern. Diese negative Korrelation zwischen Nachweis von LA-Antikörpern und Thrombosegefährdung wird damit erklärt, daß der in vivo-Effekt der LA-Antikörper nicht bekannt ist. Als mögliche Erklärung wird eine Beeinträchtigung der fibrinolytischen Aktivität (Tsakiris et al., Thromb.Haemostas. 61 (1989), 175-177; Nilsson, T.K., Löfvenberg, E., Clin.Rheumatol. 8 (1989), 58-63), verzögerte Bildung und Freisetzung von Prostacyclin (Schorer et al., Br.J.Haematol. 71 (1989), 399-407), vermehrte Freisetzung des von Willebrand-Faktors (Byron et al., Ann.Rheum.Dis. 46 (1987), 741-745) und gestörte Aktivierung des Protein C (Tsakiris et al., J.Rheumatol. 17 (1990), 785-789) berichtet. Allen diesen Erklärungsmöglichkeiten gemeinsam ist die primäre Bindung der LA-Antikörper an die Endothelzelloberfläche, was eine gestörte endotheliale Funktion zur Folge hat. Umfangreiche Untersuchungen haben jedoch gezeigt, daß diese aufgelisteten patho-physiologischen Erklärungen für einige, jedoch nicht für die große Mehrzahl der Patienten mit LA-Antikörpern zutreffen.

Der Erfindung liegt daher die Aufgabe zugrunde, ein Verfahren zu entwickeln, um Thrombose verursachende LA-Antikörper spezifisch und quantitativ zu bestimmen. Der Test sollte einfach und schnell durchführbar sein und vor allen Dingen eine hohe Spezifität in Bezug auf die Thrombosegefährdung aufweisen.

Gelöst wird diese Aufgabe erfindungsgemäß durch ein Verfahren zur Bestimmung von Lupus-anticoagulant (LA)-Antikörpern in Blut-, Plasma- oder Gewebeproben über die durch diese Antikörper bewirkte Hemmung der neutralisierenden Wirkung, die aktiviertes Protein C auf das Blutgerinnungssystem ausübt, bei dem man zu der Probe eine definierte Menge aktiviertes Protein C zusetzt, nach Inkubation die verbleibende Menge eines physiologischen Substrats von Protein C oder die Protein S-Aktivität in der Probe nach an sich bekannten Methoden bestimmt und die Menge an vorhandenen LA-Antikörpern über den Vergleich mit einem keine LA-Antikörper enthaltenden Standard errechnet. Dieses Verfahren baut auf der bekannten Reaktion auf, daß aktiviertes Protein C in Abhängigkeit von Phospholipiden insbesondere Faktor VIIIa oder Vaktor Va proteolytisch spaltet. Um das Problem unterschiedlicher Konzentrationen an Protein C in verschiedenen Testproben zu umgehen, wird aktiviertes Protein C der Testprobe zugesetzt und die Hemmung (Neutralisation) der antikoagulatorischen Aktivität des aktivierten Protein C an physiologischen Substraten in Anwesenheit von Anti-Phospholipidantikörpern bestimmt. In einem Patientenkollektiv mit nachgewiesenen LA-Antikörpern korreliert die Rate der Hemmung (Neutralisation) der aktivierten Protein C-Aktivität nicht mit der Konzentration der gemessenen LA-Antikörper. Mit dem erfindungsgemäßen Test kann jedoch gezeigt werden, daß die Rate der Hemmung der Protein-C-Aktivität im gleichen Patientenkollektiv gut mit der Inzidenz thrombotischer Ereignisse korreliert.

Als Substrat des aktivierten Protein C kann im Rahmen der Erfindung jedes physiologische Substrat des aktivierten Protein C, also insbesondere Faktor VIIIa und Faktor Va, verwendet werden. Auch läßt sich die LA-Antikörperwirkung über ein System zur Bestimmung der Protein S-Aktivität erfassen. Zur Detektion der proteolytischen Wirkung des aktivierten Protein C auf die Faktoren VIIIa und Va eignen sich insbesondere Gerinnungstests, vorzugsweise unter Verwendung chromogener Substrate, und auch monospezifische Antikörper.

Besonders bevorzugt wird in diesem Test die Detektion der Aktivität des aktivierten Protein C über die Proteolyse des aktivierten Faktors VIIIa durchgeführt, obwohl dies nicht erfindungswesentlich ist und es allein darauf ankommt, die Hemmung der Phospholipid-abhängigen Reaktion zwischen aktiviertem Protein, Protein S und physiologischem Subtrat zu erfassen. Im Rahmen des erfindungsgemäßen Verfahrens wird also die Hemmung der enzymatischen Wirkung des aktivierten Protein C in Abhängigkeit von LA-Antikörpern bestimmt.

Im Rahmen des erfindungsgemäßen Verfahren wird vorzugsweise gereinigtes Protein C verwendet, und zwar insbesondere rekombinant hergestelltes Protein C.

Die Aktivierung des Protein C wird im Rahmen des erfindungsgemäßen Verfahrens vorzugsweise durch Thrombin oder Schlangengift bewirkt, und zwar insbesondere durch an eine Festphase gekoppeltes Thrombin oder Schlangengift. Die Kopplung an CNBr-activated Sepharose 4B erfolgte dabei nach der vom Hersteller angegebenen Vorschrift: "Methods for coupling ligands to CNBr-activated Sepharose 4B" in Affinity Chromatography - prinziples and methods - Pharmacia LKB Biotechnology, Uppsala, Sweden, Informationsbroschüre.

Zur Bestimmung der Thrombose-verursachenden LA-Antikörper in Plasmafraktionen bzw. Zellextrakten oder Punktaten mit niedriger Aktivität eines Substrats für aktiviertes Protein C kann eine Vorbehandlung der Proben notwendig sein. Dabei wird dem Patienten-Plasma ein physiologisches Substrat für Protein C, insbesondere Faktor VIIIa oder Faktor Va zugesetzt.

Besonders bevorzugt ist es hierbei, markiertes und insbesondere mit einem Farbstoff, z.B. Fluoreszenzfarbstoff, markiertes Substrat, insbesondere Faktor Va, Faktor VIIIa zuzusetzen.

Es kann auch vorteilhaft sein, der Probe neben aktiviertem Protein C auch Protein S zuzusetzen, so daß eine ausreichende Komplexbildung sichergestellt ist.

In einer besonders bevorzugten Ausführungsform wird das zu testende Plasma mit aktiviertem Protein C inkubiert und anschließend je nach zu bestimmendem Substrat ein entsprechendes Mangelplasma, also Faktor VIII - oder Faktor V - Mangelplasma zur Bestimmung der verbliebenen Faktor-VIIIa-Aktivität in einem Gerinnungstest zugesetzt.

Durch die Erfindung wird eine einfache, rasch durchführbare Bestimmung der neutralisierenden Aktivität der antikoagulatorischen Aktivität des APC ermöglicht, die für die Diagnose und Therapie von Erkrankungen vorteilhaft angewendet werden kann. Die im Zusammenhang mit der Entstehung von LA-Antikörpern auftreten, was wiederum als Verfahren zur Diagnose einer Prädisposition für thrombotische Eigenschaften oder von krankheitsbedingter Thrombosegefahr sowie zur Überwachung einer Therapie ein weiterer Gegenstand der vorliegenden Erfindung ist. Das erfindungsgemäße Verfahren erlaubt die Bestimmung der LA-Antikörper ohne Isolierung der Antikörper aus dem Plasma. Die Bestimmung kann direkt im Blut, Plasma oder Punktat des Patienten durchgeführt werden und ist dementsprechend schnell und im Vergleich zu anderen Testverfahren billig.

Ein wiederum weiterer Gegenstand der Erfindung ist die Verwendung eines Reagenzes, welches für die Bestimmung der Thrombose-verursachenden LA-Antikörper geeignet ist, welches aktiviertes Protein C, kombiniert mit den Bestandteilen eines Gerinnungstests und vorzugsweise eines Faktor VIIIa-Aktivitätstests enthält. Die folgenden Beispiele erläutern die Erfindung in Verbindung mit den beigefügten Zeichnungen weiter. Hierbei stellen dar:
- Figur 1:: Graphische Darstellung der Gerinnungszeiten eines Faktor VIII-Aktivitätstests aufgetragen gegen die Konzentration an aktiviertem Protein C.
- Figur 2:: Graphische Darstellung der APC-Antikoagulant-Neutralisationsaktivität (APC-ANA) von Immunglobulinen, isoliert aus Plasma von einem Patienten, im Vergleich zu normalen Immunglobulin. Dargestellt sind die gemessenen Gerinnungszeiten und die analogen APC-ANA-Werte. Aufgetragen ist die neutralisierende Aktivität, bestimmt nach der in Beispiel 1 beschriebenen Methodik, in Abhängigkeit von der Immunglobulinkonzentration.

### BEISPIEL 1:

### Bestimmung der APC-induzierten Faktor-VIII-Inaktivierung im Normalplasma

### A) Herstellung von aktiviertem Protein C als standardisiertes Enzym in dem Reagenz

Thrombin, an Sepharose gekoppelt, wird eine Stunde bei 37°C mit gereinigtem Protein C inkubiert. Das Verhältnis von Thrombin zu Protein C beträgt 1:30 (mol/mol). Die Menge des entstandenen aktivierten Protein C (APC) wird mit einem Protein C-Aktivitätstest bestimmt, um sicher zu sein, daß die eingesetzte Protein C-Menge völlig aktiviert wurde. Es hat sich der Protein C-Aktivitätstest unter Verwendung von einem chromogenen Substrat bewährt. APC kann bis zur Verwendung im Test lyophilisiert aufgehoben weren.

### B) Durchführung des Tests

### 1. Zweistufen-Test

50 µl Probelösung werden zu 200 µl APC hinzugefügt, so daß eine Endkonzentration an APC in der Mischung zwischen 0,1 und 1,5 µM erreicht wird. APC und Probelösung werden gemischt und 2 Minuten bei 37°C inkubiert. 100 µl der Mischung werden anschließend mit 100 µl Faktor VIIIa-Mangelplasma und 100 µl eines Reagenzes zur Bestimmung der partiellen Thromboplastin-Zeit gemischt. Nach einer abermaligen Inkubationszeit, wie vorgeschrieben für die Bestimmung der Faktor VIIIa-Aktivität, wird durch Zugabe von 100 µl Calciumchlorid (25 mM) gestartet. Die Zeit von Beginn der Zugabe der Calciumchloridlösung bis zum Eintritt eines Gerinnsels wird visuell oder unter Verwendung eines Automaten registriert.

### 2. Einstufen-Test

20 µl Probelösung werden zu 80 µl APC hinzugefügt, so daß eine Endkonzentration in der Mischung von APC und Probe zwischen 0,01 und 1,5 µM erreicht wird. APC und Probelösung werden gemischt und 2 Minuten bei 37°C inkubiert. Hieran anschließend werden der Reaktionsmischung 100 µl Faktor VIII-Mangelplasma und 100 µl eines Reagenzes zur Bestimmung der partiellen Thromboplastin-Zeit hinzugefügt und gemischt. Nach einer abermaligen Inkubationszeit, wie vorgeschrieben für die Bestimmung der Faktor VIII-Aktivität, wird durch Zugabe von 100 µl Calciumchlorid (25 mM) gestartet. Die Zeit von Beginn der Zugabe der Calciumchloridlösung bis zum Eintritt eines Gerinnsels wird visuell oder unter Verwendung eines Automaten registriert.

### BEISPIEL 2:

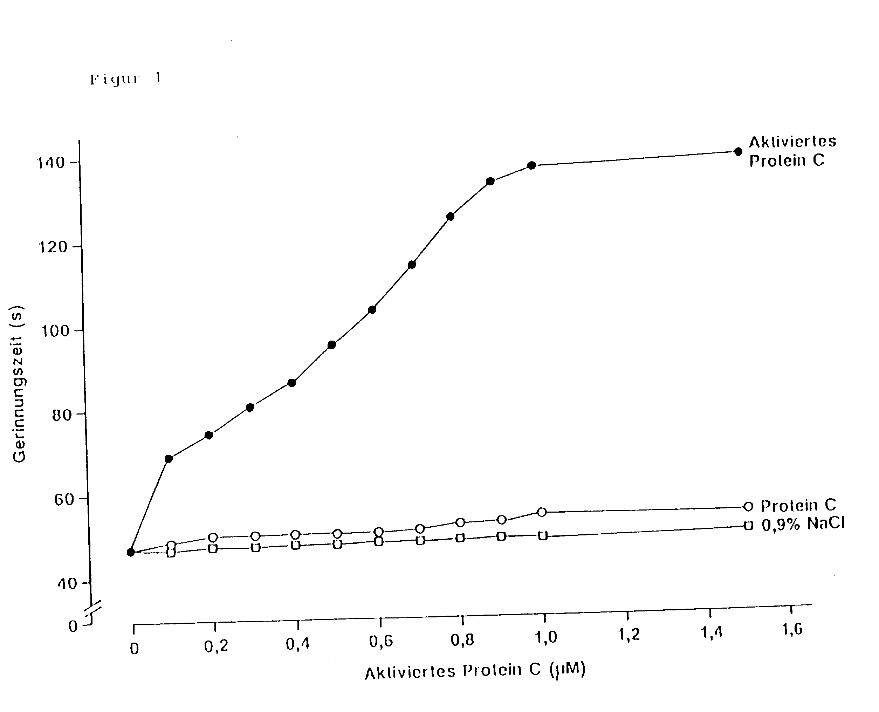

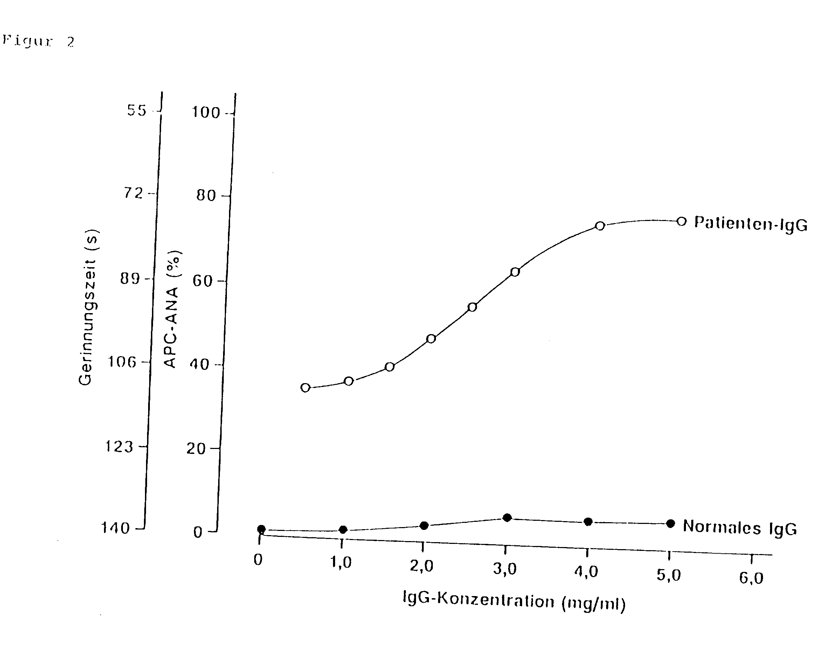

Bestimmung der APC-Anticoagulant-Neutralisationsaktivität (APC-ANA) von Immunglobulinen, isoliert aus humanem Patientenplasma. Um die Spezifität des Testes darzulegen, wurde die Immunglobulinfraktion aus Patientenplasma isoliert und in einer Dosis-Wirkungs-Kurve der Effekt der Immunglobuline in dem oben beschriebenen Test bestimmt. Immunglobuline aus Patientenplasma und normale Immunglobuline wurden mit APC 5 Minuten lang bei 37°C inkubiert. Anschließend wurde Faktor VIII-Mangelplasma, Reagenz zur Bestimmung der partiellen Thromboplastin-Zeit und nach einer abermaligen Inkubationszeit Calciumchlorid hinzugefügt. Figur 2 zeigt, daß mit steigender IgG-Konzentration an Patienten-IgG es zu einer vermehrten Hemmung (Neutralisation) der APC-antikoagulatorischen Aktivität kommt. Null % Neutralisationsaktivität stellt die maximale Proteolyse von aktiviertem Faktor VIIIa dar. Im Test werden Gerinnungszeiten von etwa 140 s gemessen (siehe Figur 1). Hundert % Neutralisationsaktivität stellt die 100 %ige Neutralisierung der APC-antikoagulatorischen Aktivität dar, die einer Gerinnungszeit entspricht, wenn nicht-aktiviertes Protein C oder physiologische Kochsalzlösung antstelle von aktiviertem Protein C im Testansatz eingesetzt werden (Figur 1).

### BEISPIEL 3:

Bestimmung der APC-Anticoagulant-Neutralisationsaktivität (APC-ANA) in humanen Plasmaproben. Um die Neutralisationsaktivität im Normalplasma (gepooltes Plasma von ca. 20 gesunden Blutspendern) und im Patientenplasma zu bestimmen, wird in dem in Beispiel 1 geschilderten Versuchsansatz als Probelösung ein Poolplasma oder das Patientenplasma eingesetzt. Als Testergebnis wird für das normale Poolplasma eine Gerinnungszeit von etwa 140 s bei Verwendung von aktiviertem Protein C in einer Konzentration von 1 µM gemessen (Figur 1 und Tabelle 1). Wird anstelle des Normalplasmas Patientenplasma verwendet, so reduziert sich die Gerinnungszeit auf etwa 70 s (Tabelle 1). Wird dasselbe Patientenplasma mit nicht-aktiviertem Protein C inkubiert, so wird eine Gerinnungszeit ermittelt, die identisch ist mit dem Wert nach Inkubation von Patientenplasma mit physiologischer Kochsalzlösung (Tabelle 1). Die Gerinnungszeit ist unter Umständen ein wenig länger als bei Normalplasma, da LA-Antikörper nicht nur APC-neutralisierende Aktivität aufweisen können, sondern noch mit weiteren Schritten in dem Gerinnungstest reagieren. Aus diesem Grunde wird zur Bestimmung der APC-Anticoagulant-Neutralisationsaktivität im Patientenplasma die Differenz zwischen dem Wert gewählt, wenn man Patientenplasma mit APC inkubiert und dem Wert nach Inkubation von Patientenplasma mit nicht aktiviertem Protein C (Tabelle 1).

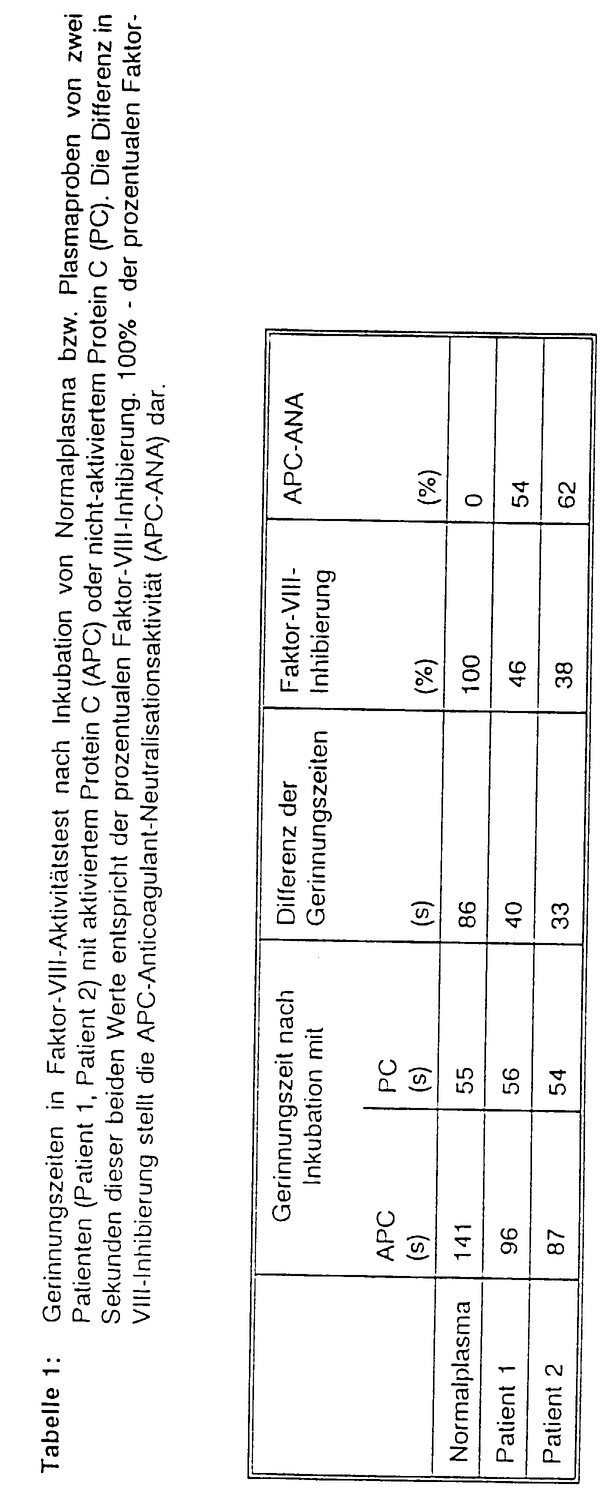

## Patentansprüche

1. Verfahren zur Bestimmung von Lupus-anticoagulant (LA)-Antikörpern in Blut-, Plasma- oder Gewebeproben über die durch diese Antikörper bewirkte Hemmung der neutralisierenden Wirkung, die aktiviertes Protein C auf das Blutgerinnungssystem ausübt, bei dem man zu der Probe eine definierte Menge aktiviertes Protein C zusetzt, nach Inkubation die verbleibende Menge eines physiologischen Substrats von Protein C oder die Protein S-Aktivität in der Probe nach an sich bekannten Methoden bestimmt und die Menge an vorhandenen LA-Antikörpern über den Vergleich mit einem keine LA-Antikörper enthaltenden Standard errechnet.

2. Verfahren nach Anspruch 1,
**dadurch gekennzeichnet**,
daß man gereinigtes Protein C verwendet.

3. Verfahren nach Anspruch 1 oder 2,
**dadurch gekennzeichnet**,
daß man rekombinant hergestelltes Protein C verwendet.

4. Verfahren nach einem der vorhergehenden Ansprüche,
**dadurch gekennzeichnet**,
daß man durch Thrombin oder Schlangengift aktiviertes Protein C verwendet.

5. Verfahren nach Anspruch 4,
**dadurch gekennzeichnet**,
daß man zur Aktivierung an eine Festphase gekoppeltes Thrombin oder Schlangengift verwendet.

6. Verfahren nach einem der vorhergehenden Ansprüche,
**dadurch gekennzeichnet,**
daß man als Protein C-Substrat Faktor Va oder VIIIa verwendet.

7. Verfahren nach Anspruch 6,
**dadurch gekennzeichnet,**
daß man die verbleibende Menge an Faktor Va oder Faktor VIIIa über einen Gerinnungstest bestimmt.

8. Verfahren nach Anspruch 7,
**dadurch gekennzeichnet,**
daß man in dem Gerinnungstest chromogene Substrate oder monospezifische Antikörper einsetzt,

9. Verfahren nach einem der vorhergehenden Ansprüche,
**dadurch gekennzeichnet,**
daß man der Probe ein physiologisches Substrat von Protein C zusetzt.

10. Verfahren nach Anspruch 9,
**dadurch gekennzeichnet,**
daß man markierte Faktoren Va und VIIIa zusetzt.

11. Verfahren nach einem der vorhergehenden Ansprüche,
**dadurch gekennzeichnet**,
daß man bei der Bestimmung der verbleibenden Menge an physiologischem Substrat oder der Protein S-Aktivität Faktor V -, VIIIa - bzw. Protein S-Mangelplasmen verwendet,

12. Verfahren nach einem der vorhergehenden Ansprüche,
**dadurch gekennzeichnet,**
daß man einen Komplex aus aktiviertem Protein C und Protein S der Probe zusetzt.

13. Verfahren nach einem der vorhergehenden Ansprüche,
**dadurch gekennzeichnet,**
daß man aktiviertes Protein C in einer Menge von 0,1 bis 1,5 µM zusetzt.

14. Verfahren zur Diagnose einer Prädisposition für thrombotische Ereignisse oder von krankheitsbedingter Thrombosegefahr sowie zur Überwachung einer Therapie, durch Bestimmung der Menge von LA-Antikörpern in Blut-, Plasma- oder Gewebeproben von Patienten gemäß dem Verfahren nach einem der Ansprüche 1 bis 13.

15. Verwendung eines Reagenzes zur Bestimmung von LA-Antikörpern in Blut-, Plasma- oder Gewebeproben,
**dadurch gekennzeichnet,**
daß es die Elemente eines üblichen Blutgerinnungstests sowie aktiviertes Protein C enthält.

## Claims

1. Method for the determination of lupus anticoagulant (LA) antibodies in blood, plasma or tissue samples by means of the inhibitory effect of these antibodies on the neutralizing effect of activated protein C on the blood coagulation system in which a defined amount of activated protein C is added to the sample, after incubation the remaining amount of a physiological substrate of protein C or the protein S activity in the sample is determined according to known methods and the amount of LA antibodies present is calculated by comparison with a standard containing no LA antibodies.

2. Method as claimed in claim 1,
**wherein**
purified protein C is used.

3. Method as claimed in claim 1 or 2,
**wherein**
protein C is used which was produced by recombinant means.

4. Method as claimed in one of the previous claims,
**wherein**
protein C activated by thrombin or snake venom is used.

5. Method as claimed in claim 4,
**wherein**
thrombin or snake venom coupled to a solid phase is used for the activation.

6. Method as claimed in one of the previous claims,
**wherein**
factor Va or VIIIa is used as the protein C substrate.

7. Method as claimed in claim 6,
**wherein**
the remaining amount of factor Va or factor VIIIa is determined by means of a coagulation test.

8. Method as claimed in claim 7,
**wherein**
chromogenic substrates or monospecific antibodies are used in the coagulation test.

9. Method as claimed in one of the previous claims,
**wherein**
a physiological substrate of protein C is added to the sample.

10. Method as claimed in claim 9,
**wherein**
labelled factors Va and VIIIa are added.

11. Method as claimed in one of the previous claims,
**wherein**
factor V-, factor VIII- or protein S-deficient plasma is used in the determination of the remaining amount of physiological substrate or protein S activity.

12. Method as claimed in one of the previous claims,
**wherein**
a complex of activated protein C and protein S is added to the sample.

13. Method as claimed in one of the previous claims,
**wherein**
activated protein C is added in an amount of 0.1 to 1.5 µM.

14. Method for diagnosing a predisposition to thrombotic events or thrombotic risk due to disease as well as for therapeutic monitoring by determining the amount of LA antibodies in blood, plasma or tissue samples of patients according to a method as claimed in one of the claims 1 to 13.

15. Use of a reagent for the determination of LA antibodies in blood, plasma or tissue samples,
**wherein**
it contains the components of a conventional blood coagulation test as well as activated protein C.

## Revendications

1. Procédé de dosage d'anticorps de l'anticoagulant lupique (anticorps de LA) dans des échantillons de sang, de plasma ou de tissu, par l'intermédiaire de l'inhibition par ces anticorps de l'activité neutralisante exercée par la protéine C sur le système de coagulation du sang, dans lequel on ajoute à l'échantillon une quantité définie de protéine C activée, on détermine par des procédés connus en soi, après l'incubation, la quantité résiduelle d'un substrat physiologique de la protéine C ou l'activité de protéine S dans l'échantillon, et on calcule la quantité d'anticorps de LA présents par comparaison avec un standard ne contenant pas d'anticorps de LA.

2. Procédé selon la revendication 1, caractérisé en ce que l'on utilise de la protéine C purifiée.

3. Procédé selon la revendication 1 ou 2, caractérisé en ce que l'on utilise de la protéine C préparée par une technique de génie génétique.

4. Procédé selon l'une quelconque des revendications précédentes, caractérisé en ce que l'on utilise de la protéine C activée par la thrombine ou du venin de serpent.

5. Procédé selon la revendication 4, caractérisé en ce que l'on utilise, pour l'activation, de la thrombine ou du venin de serpent immobilisés sur une phase solide.

6. Procédé selon l'une quelconque des revendications précédentes, caractérisé en ce que l'on utilise le facteur Va ou Villa comme substrat de la protéine C.

7. Procédé selon la revendication 6, caractérisé en ce que l'on détermine la quantité résiduelle de facteur Va ou de facteur Villa à l'aide d'un essai de coagulation.

8. Procédé selon la revendication 7, caractérisé en ce que l'on utilise dans l'essai de coagulation des substrats chromogènes ou des anticorps monospécifiques.

9. Procédé selon l'une quelconque des revendications précédentes, caractérisé en ce que l'on ajoute un substrat physiologique de la protéine C à l'échantillon.

10. Procédé selon la revendication 9, caractérisé en ce que l'on ajoute des facteurs Va et Villa marqués.

11. Procédé selon l'une quelconque des revendications précédentes, caractérisé en ce que l'on utilise, lors de la détermination de la quantité résiduelle de substrat physiologique ou de l'activité de protéine S, des plasmas déficients en facteur V, en facteur VIII ou en protéine S.

12. Procédé selon l'une quelconque des revendications précédentes, caractérisé en ce que l'on ajoute à l'échantillon un complexe de protéine C activée et de protéine S.

13. Procédé selon l'une quelconque des revendications précédentes, caractérisé en ce que l'on ajoute de la protéine C activée en une quantité de 0,1 à 1,5 µM.

14. Procédé de diagnostic d'une prédisposition à des phénomènes thrombotiques ou d'un risque de thrombose dû à une maladie, et de suivi d'une thérapie, par détermination de la quantité d'anticorps de LA dans des échantillons de sang, de plasma ou de tissu de malades par le procédé selon l'une des revendications 1 à 13.

15. Utilisation d'un réactif pour le dosage d'anticorps de LA dans des échantillons de sang, de plasma ou de tissu, caractérisé en ce qu'il contient les éléments d'un essai classique de coagulation du sang et de la protéine C activée.
